# EUROPEAN PATENT SPECIFICATION

(11) **EP 0 325 178 B1**
(45) Date of publication and mention of the grant of the patent: **12.05.1993**
(21) Application number: 89100589.4
(22) Date of filing: 13.01.1989
(51) Int. Cl.: B01L 7/00

(54) **Apparatus for heating a sample within a vacuum chamber**
Vorrichtung zum Erwärmen einer Probe in einer Vakuumkammer
Dispositif de chauffage d'échantillon dans une chambre à vide

(30) Priority: 20.01.1988 JP 11319/88
(43) Date of publication of application: 26.07.1989
(73) Proprietor: HORIBA, LTD., Minami-ku Kyoto (JP)
(72) Inventor: Motoi, Yoshihiko, Nishikyo-ku Kyoto (JP); Yamamoto, Naoki, Saitama prefecture (JP); Takano, Yukio, Tokyo (JP)
(74) Representative: TER MEER STEINMEISTER & PARTNER GbR

(56) References cited:
- GB-A- 1 430 367
- US-A- 3 915 118
- POLYTECHNISCH TIJDSCHRIFT: WERKTUIGBOW, vol. 30, no. 7, 1975, pages 223-224; H. KUSTER et al.: "Electrisch bediende grijpinrichting voor toepassing in vacuüm"

## Description

The present invention relates to an apparatus for heating a sample within a vacuum chamber. Such an apparatus may for example form part of an analyzer having a vacuum chamber in which the sample to be analysed may be heated.

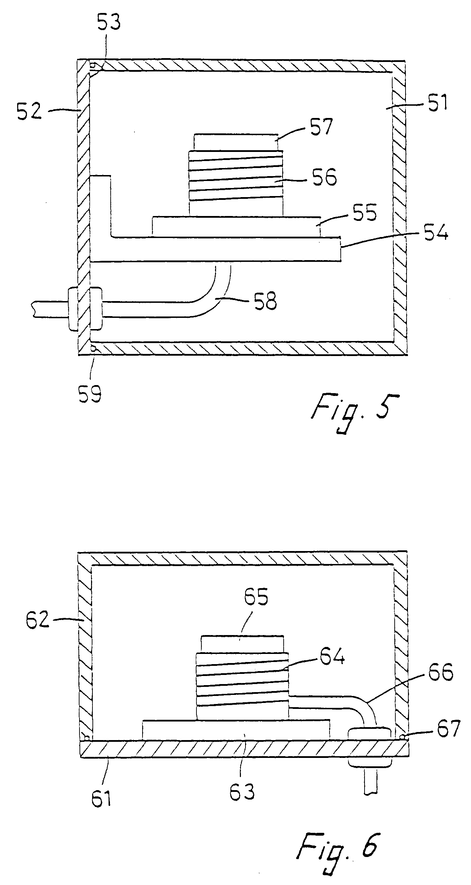

An example of a conventional apparatus of this type is shown in figure 5.

A vacuum chamber 51 of, for example, an analyzer is provided with an access opening 53 which serves for placing a sample within the vacuum chamber and for removing the sample from the vacuum chamber, respectively. The opening 53 is adapted to be vacuum-tightly closed by means of a cover 52. A bracket 54 is fixedly mounted on an inner surface of the cover 52. A heating block 56 comprising a heater and a temperature-measuring element is mounted on a stage 55 mounted on said bracket 54. An upper portion of the heating block 56 serves as a sample table 57. A wiring 58 for connecting the heating block 56 to external equipment passes through the cover 52. Reference numeral 59 designates a packing mounted on a mouth portion of the axis opening 53.

When, in this apparatus, a sample is to be mounted on the sample table 57, the cover 52 is separated from the vacuum chamber 51, and the whole assembly including the heating block 56 and the stage 55 is taken out of the vacuum chamber 51. Then, the sample is mounted on the sample table 57.

Afterwards, the whole assembly including the heating block 56 is again inserted into the vacuum chamber 51 through the access opening 53 thereof, and the access opening 53 is again closed by means of the cover 52. Finally, the vacuum chamber 51 is evacuated.

Also, when the sample is to be removed from the sample table 57, the whole assembly including the heating block 56 is taken out of the vacuum chamber 51 through the access opening 53 upon removing the cover 52. Accordingly, when the sample is mounted on or detached from the sample table 57, the vacuum chamber 51 is opened to the atmosphere.

Another example of a conventional heating apparatus is shown in figure 6.

Referring to figure 6, reference numeral 61 designates a bottom plate on which a bell jar 62 is placed. Reference numeral 63 designates a stage mounted on the bottom plate 61. A heating block 64 comprising a heater and a temperature-measuring element is mounted on said stage 63. An upper portion of the heating block 64 serves as a sample table 65. A wiring 66 for the heating block 64 passes through the bottom plate 61. Reference numeral 67 designates a packing mounted on the bell jar 62.

In this apparatus, the sample is mounted on and detached from the sample table 65, respectively, by separating the bell jar 62 from the bottom plate 61 to expose the sample table 65.

The apparatus shown in figure 5 has a drawback in that the whole assembly including the heating block 56, the stage 55 and the bracket 54 must be taken out of the vacuum chamber 51 each time a sample is to be mounted and removed, respectively. Accordingly, it requires much time and labor to replace the sample by a new one. Further, since the vacuum chamber 51 is opened to the atmosphere while the assembly including the heating block and the cover is removed, additional time and labor is required for relieving the vacuum before the sample is removed and for evacuating the vacuum chamber again after the new sample has been mounted.

Similarly, in case of the apparatus shown in figure 6, it is necessary to remove the bell jar 62 each time a sample has to be mounted and detached, respectively. When the bell jar 62 is separated from the botton plate 61, it is open to the atmosphere. Accordingly, it is necessary to evacuate the bell jar 62 again after a new sample has been mounted.

None of the above-descripted conventional apparatus permits to detach the heating block from the stage on which it is mounted and to disconnect the heater from the wiring which is vacuum-tightly passed through the cover 52 or the bottom plate 61, respectively, of the vacuum chamber.

US-A-3 915 118 discloses an apparatus for heating a sample within a vacuum chamber of a scanning electron microscope. said apparatus having the features indicated in the pre-characterizing part of claim 1. This apparatus is provided with a transfer device with which a sample holder can be detached from a stage provided within the vacuum chamber and can be transferred to a preliminary exhaust chamber which is connected with the vacuum chamber through a gate valve. The transfer device comprises an operating shaft which is longitudinally slidably mounted in a wall of the preliminary exhaust chamber opposite to the gate valve, and which has a threaded end portion which is threaded into a bore of the sample holder so that the sample holder can be moved back and forth by longitudinally sliding the operating shaft. Since the preliminary exhaust chamber can be separated from the vacuum chamber while the sample holder is positioned within the preliminary exhaust chamber, it is not necessary to relieve the vacuum in the vacuum chamber when the sample is mounted or detached. When the sample holder has been transferred onto the stage within the vacuum chamber, it remains connected to the operating shaft while the sample is being inspected.

It is an object of the present invention to provide an apparatus of the type indicated in the pre-characterizing part of claim 1 in which the operating shaft can easily be disconnected from the sample holder and can be retracted after the sample holder has been mounted on the stage, and the operating shaft can easily be re-connected to the sample holder in order to remove the same from the vacuum chamber.

This object is achieved with the features indicated in claim 1.

Further embodiments of the invention are indicated in the dependent claims.

A preferred embodiment of the invention will be described below with reference to figures 1 to 4 of the drawings in which
- Fig. 1: is a sectional view of a heating apparatus embodying the invention;
- Fig. 2: is an enlarged plan view showing a sample holder and a stage of the heating apparatus according to fig. 1;
- Fig. 3: is an enlarged front view showing the sample holder and the stage;
- Fig. 4: is a side view showing the sample holder and the stage; and
- Figs. 5 and 6: are sectional views showing two different examples of conventional heating apparatus.

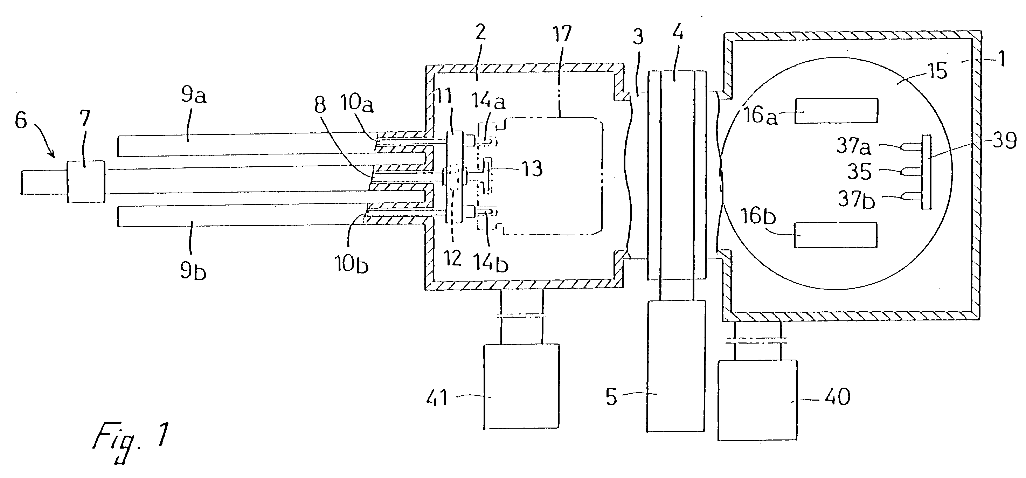

Referring to figs. 1 to 4, reference numeral 1 designates a vacuum chamber, and reference numeral 2 designates a preliminary exhaust chamber. A communicating portion 3 connecting the vacuum chamber 1 to the preliminary exhaust chamber 2 is adapted to be opened and closed by means of a gate valve 4 which is operated by means of a cylinder 5.

A magnetic coupling type transfer device 6 is disposed in the preliminary exhaust chamber 2 and is adapted to transfer an operating shaft 8 from the interior of the preliminary exhaust chamber 2 to the interior of the vacuum chamber 1 or vice versa by sliding a dial 7 of a transfer device 6. Further, the operating shaft 8 may be rotated about its longitudinal axis by rotating the dial 7.

Reference numerals 9a, 9b designate cylindrical rectilinear propagation guides connected to an end wall of the preliminary exhaust chamber 2 and arranged on both sides of the transfer device 6 substantially in parallel thereto. Guide rods 10a, 10b are slidably inserted in the cylindrical guides 9a and 9b, respectively. A holding member 11 is mounted on the pointed ends of the guide rods 10a, 10b. The operating shaft 8 passes through the holding member 11 and is held rotatably and unslidably with respect to the holding member 11 in a bearing 12.

Thus, upon sliding the operating shaft 8 by operating the dial 7, the guide rods 10a, 10b are simultaneously slid through the guides 9a, 9b, whereby the holding member 11 is moved linearly without being rotated.

Reference numeral 13 designates a rod-like hook fixedly mounted on the pointed end of the operating shaft 8 so as to meet at right angles with the operating shaft 8. Reference numerals 14a, 14b designate holding pins projecting from the holding member 11 on both sides of the operating shaft 8. The holding pins 14a, 14b are substantially coaxial with the guide rods 10a, 10b, but they may be disposed at other positions different from those of the guide rods 10a, 10b.

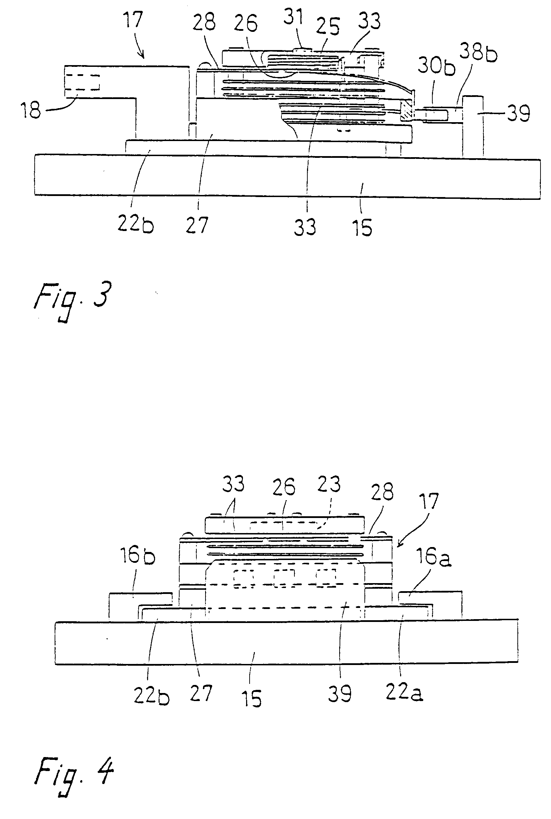

A stage 15 is disposed within the vacuum chamber 1 and is provided with a pair of fitting members 16a, 16b having a reverse letter-L-shaped section. The fitting members 16a, 16b are fixedly mounted on a surface of the stage 15 spaced apart from and in parallel with each other and with gap portions thereof facing each other, as is shown in figure 4.

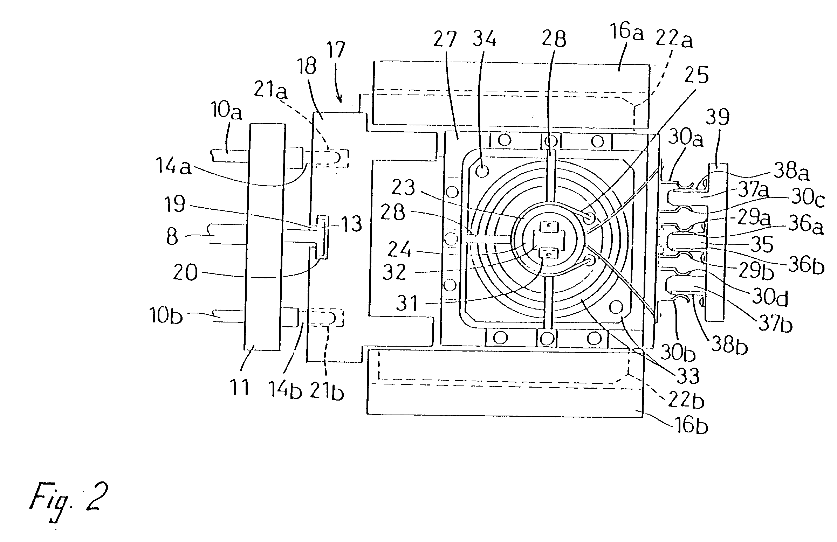

A sample holder 17 (figures 1 and 2) is provided at one end thereof with a support member 18. The support member 18 is formed with an engaging hole 20 having a narrow opening 19. The hook 13 of the operating shaft 8 may be inserted through said narrow opening 19 by moving the operating shaft 8 longitudinally and may then be engaged in the engaging hole 20 by rotating the operating shaft 8 by about 90°.

Reference numerals 21a, 21b designate inserting holes formed in end faces of the sample holder 17 on both sides of said opening 19, the distances from these inserting holes 21a, 21b to said opening 19 being equal to the distances from the hook 13 to the holding pins 14a, 14b, so that the holding pins 14a, 14b can be inserted into the inserting holes 21a, 21b.

Reference numerals 22a, 22b designate engaging plates disposed on both sides of the sample holder 17 and adapted to be inserted between and engaged by said fitting members 16a, 16b, so that the sample holder 17 may be fitted on the stage 15.

Reference numeral 23 designates an insulator provided with a sample table 24 buried in a recessed central portion of the insulator. A heater 25 is provided on the circumferential surface of the insulator, and a temperature-measuring element 26, such as a thermocouple or a platinum resistance-measuring element is provided on the backside or lower side of said insulator. A plurality of support plates 28 are mounted with their respective outer ends on a frame-like fitting table 27 which is fixedly mounted on an upper surface of the sample holder 17. The insulator 23 is mounted on the projecting inner end portions of said support plates 28 and is thus held freely in an internal space of the fitting table 27.

Reference numerals 29a, 29b designate connectors with which said heater 25 is connected, reference numerals 30a, 30b designate connectors with which the temperature-measuring element 26 is connected, and reference numerals 30c, 30d designate supplementary connectors disposed opposite to the connecters 30a, 30b. The connectors 29a, 29b, 30a, 30b, 30c, 30d are fixedly mounted on the fitting table 27 at an end portion of the sample holder 17 opposite to the support member 18.

Reference numeral 31 designates a sample-fixing member disposed in the sample table 24, reference numeral 32 designates a sample, and reference numeral 33 designates ring-shaped heat-reflecting plates disposed below and on side portions of the insulator 23 for reflecting heat irradiated from the circumferential surface and the back surface of the insulator 23 so as to improve the heating efficiency. Reference numeral 34 designates support rods for the heat-reflecting plates 33.

Fixed connectors 35 are inserted between the connectors 29a, 29b and are provided with terminals 36a, 36b on a surface brought into contact with the connectors 29a, 29b, respectively. Fixed connectors 37a, 37b are inserted between the connector 30a and the supplementary connector 30c and between the connector 30b and the supplementary connector 30d, respectively, and are provided with terminals 38a, 38b brought into contact with the connectors 30a, 30b.

The fixed connectors 35, 37a, 37b project from a side surface of a fitting plate 39 standing on a surface of the stage 15 and come into engagement with the connectors 29a, 29b, 30a, 30b, 30c, 30d, when the sample holder 17 is fitted on the stage 15 by inserting the engaging plates 22a, 22b between the fitting members 16a, 16b.

The terminals 36a, 36b, 38a, 38b of the fixed connectors are connected with respective lead wires (not shown) which are taken out of the vacuum chamber 1.

A vacuum pump 40 is connected with the vacuum chamber 1, and a vacuum pump 41 is connected with the preliminary exhaust chamber 2.

The preliminary exhaust chamber 2 is provided with a mouth (not shwon) through which the sample holder 17 may be put into or taken out of the interior of the preliminary exhaust chamber. The mouth is closable by means of a cover (not shown).

The operation of the apparatus according to the invention will be described below.

It is assumed that the sample holder 17 has been removed from the apparatus, the gate valve 4 is closed and a vacuum is maintained in the vacuum chamber 1. The sample 32 is fixedly mounted on the sample table 24 of the sample holder 17 by means of the sample-fixing member 31. The sample holder 17 is put into the preliminary exhaust chamber 2, and the dial 7 is operated to slide the operating shaft 8, whereby the hook 13 is inserted through the opening 19 into the engaging hole 20, and the holding pins 14a, 14b are inserted into the inserting holes 21a, 21b of the sample holder 17.

Subsequently, the dial 7 is rotated to rotate the hook 13 by almost 90°, so that the hook 13 is engaged in the engaging hole 20 and the sample holder 17 is mounted on the transfer device 6 (figures 1 and 2). Then, the preliminary exhaust chamber 2 is closed tightly and evacuated by means of the vacuum pump 41.

After the preliminary exhaust chamber 2 has been evacuated, the gate valve 4 is opened by actuating the cylinder 5, and the dial 7 is slid to insert the operating shaft 8 into the vacuum chamber 1 together with the guide rods 10a, 10b. In this way, the sample holder 17 is transferred along the surface of the stage 15 and the engaging plates 22a, 22b are engagingly inserted into the gap between the fitting members 16a, 16b, so that the sample holder 17 is mounted on the stage 15.

During the end phase of the sliding movement of the sample holder 17, the fixed connector 35 is inserted between the connectors 29a and 29b, and the fixed connectors 37a, 37b are inserted between the connectors 30a, 30b and the supplementary connectors 30c, 30d, respectively, to connect the connectors 29a, 29b, 30a, 30b to the terminals 36a, 36b, 38a, 38b.

After the sample holder 17 has been mounted on the stage 15 in this way, the operating shaft 8 is rotated by operation of the dial 7. The hook 13 is set to be parallel to the opening 19, and then the operating shaft 8 is retracted to separate the hook 13 and the holding pins 14a, 14b from the sample holder 17.

The sample 32 mounted on the sample table 24 of the sample holder 17 is heated by applying a voltage to the heater 25 through the terminals 36a, 36b and the connectors 29a, 29b and heating the sample table 24 mounted on the insulator 23 in a burried manner. The temperature control is carried out on the basis of a signal transmitted from the temperature-measuring element 26 mounted on the rear surface of the insulator 23 through the connectors 30a, 30b and the terminals 38a, 38b.

As will be understood from the above description, the heating apparatus according to the invention has the advantage that is is not necessary to expose the vacuum chamber to the atmosphere when a sample is mounted therein or is removed therefrom, and the sample holder can easily and efficiently be mounted in the vacuum chamber and taken out of the vacuum chamber by means of the transfer device. Further, when the sample holder is mounted on the transfer device and positioned within the preliminary exhaust chamber and the preliminary exhaust chamber is evacuated, water and similar contaminants adhered to the sample holder can be removed in the preliminary exhaust chamber, so that a proper vacuum is maintained in the vacuum chamber when the sample holder is transferred thereto. Accordingly, the heating of the sample can be carried out without delay and under optimal vacuum conditions.

In addition, the connectors mounted on the sample holder and the fixed connectors disposed within the vacuum chamber are automatically engaged with each other and disengaged from each other due to the movement of the sample holder when the latter is mounted on the stage and detached therefrom, respectively, so that a special labor for connecting and disconnecting the heater is not required.

Since, further, the heater 25 is mounted on the circumferential surface of the insulator 23 and the sample table 24 is mounted on the insulator 23 in a buried manner, the sample table 24 can be efficiently heated to efficiently heat the sample to the desired temperature.

On the other hand, the apparatus according to the invention may also be used in cases where the sample need not be heated. In these cases, the sample holder may be replaced by another sample holder which is not provided with a heater and a temperature-measuring element but can be mounted on the stage 15 by means of the transfer device 6 in the above described manner.

Further, the connectors mounted on the sample holder and the fixed connectors disposed in the vacuum chamber may have a modified construction so that they can simultaneously be used to mount the sample holder 17 on the stage 15.

The construction of the transfer device may be modified in various ways. For example, an O-ring seal type transfer device may be used instead of the above described magnetic coupling type transfer device. In place of the hook 13 of the operating shaft 8, a screw shaft can be used. In this case, the sample holder 17 is provided with a threaded hole.

## Claims

1. An apparatus for heating a sample (32) within a vacuum chamber (1) of an analyser or the like, comprising
- a sample stage (15) disposed within the vacuum chamber,
- a sample holder (17) disposed on the stage and being provided with a heater (25),
- a preliminary exhaust chamber (2) connected with the vacuum chamber (1) through a gate valve (4),
- a transfer device (6) comprising an operating shaft (8) which is connectable at one end thereof with said sample holder (17) and is longitudinally slidable for detaching the sample holder (17) from the stage (15) and moving it to the preliminary exhaust chamber (2) and for moving the sample holder back into the vacuum chamber and mounting it on the stage,
- the sample holder being provided with connector means (29a,29b) which cooperate with fixed connector means (35) mounted within the vacuum chamber for electrically connecting the heater (25) when the sample holder is mounted on the stage,
**characterized** in that said operating shaft (8) is connected to the sample holder (17) through a hook (13) extending at right angles with respect to the operating shaft (8) and being insertable into an engaging hole (20) of the sample holder (17) through a narrow opening (19), and the operating shaft (8) is rotatable about its longitudinal axis for establishing and cancelling engagement between said hook (13) and the sample holder.

2. An apparatus as set forth in claim 1, wherein the sample holder (17) is provided with a temperature-measuring element (26) and with connector means (30a,30b) which cooperate with fixed connector means (37a,37b) mounted within the vacuum chamber (1) for connecting the temperature-measuring element when the sample holder is mounted on the stage.

3. An apparatus as set forth in claim 1 or 2, wherein said operating shaft (8) is received in a tubular member projecting outwardly from a wall of the preliminary exhaust chamber (2) and a dial (7) is slidable on said tubular member, said operating shaft (8) being drivingly coupled to said dial (7) by magnetic coupling.

4. An apparatus as set forth in any of the preceding claims, wherein said operating shaft (8) is held rotatably and non-slidably in a holding member (11) which is held non-rotatably with respect to the preliminary exhaust chamber (2) by means of guide rods (10a,10b) and is connectable to the sample holder (17) through holding pins (14a, 14b) which extend in parallel to the operating shaft (8).

5. An apparatus as set forth in any of the preceding claims, wherein said connector means (29a,29b,35;30a,30b,37a,37b) form plug-type connectors, said fixed connector means (35,37a,37b) being disposed on the stage (15) in a position opposite to the gate valve (4).

6. An apparatus as set forth in any of the preceding claims, wherein said sample holder comprises a sample table (24) buried in a central portion of an insulator (23), said heater (25) being provided on a circumferential surface of said insulator (23) for heating the sample table (24).

## Patentansprüche

1. Vorrichtung zum Erwärmen einer Probe (32) in einer Vakuumkammer (1) eines Analysegerätes oder dergleichen, mit
- einem in der Vakuumkammer angeordneten Proben-Gestell (15),
- einem auf dem Gestell angeordneten und mit einer Heizung (25) versehenen Probenhalter (17),
- einer Vorvakuumkammer (2), die mit der Vakuumkammer (1) über einen Sperrschieber (4) verbunden ist,
- einer Transfereinrichtung (6) mit einer Betätigungswelle (8), die an ihrem einen Ende mit dem Probenhalter (17) verbunden ist und in Längsrichtung verschiebbar ist, um den Probenhalter (17) von dem Gestell (15) zu lösen und in die Vorvakuumkammer (2) zu überführen und um den Probenhalter zurück in die Vakuumkammer zu überführen und auf dem Gestell zu montieren,
- wobei der Probenhalter mit Anschlußmitteln (29a,29b) versehen ist, die mit in der Vakuumkammer montierten festen Anschlußmitteln zusammenwirken, um die Heizung (25) anzuschließen, wenn der Probenhalter auf dem Gestell montiert ist,
dadurch **gekennzeichnet,** daß die Betätigungswelle (8) mit dem Probenhalter (17) durch einen Haken (13) verbunden ist, der rechtwinklig in bezug auf die Betätigungswelle (8) orientiert und durch eine enge Öffnung (19) in ein Eingriffsloch (20) des Probenhalters (17) einführbar ist, und daß die Betätigungswelle (8) um ihre Längsachse drehbar ist, um den Eingriff zwischen dem Haken (13) und dem Probenhalter herzustellen und aufzuheben.

2. Vorrichtung nach Anspruch 1, bei der der Probenhalter (17) mit einem Temperatur-Meßelement (26) und mit Anschlußmitteln (30a,30b) versehen ist, die mit in der Vakuumkammer (1) montierten festen Anschlußmitteln (37a,37b) zusammenwirken, um das Temperatur-Meßelement anzuschließen, wenn der Probenhalter auf dem Gestell montiert ist.

3. Vorrichtung nach Anspruch 1 oder 2, bei der die Betätigungswelle (8) in einem rohrförmigen Teil aufgenommen ist, das nach außen von einer Wand der Vorvakuumkammer (2) vorspringt, und eine Scheibe (7), die durch magnetische Kopplung mit der Betätigungswelle (8) in Antriebsverbindung steht, auf dem rohrförmigen Teil verschiebbar ist.

4. Vorrichtung nach einem der vorstehenden Ansprüche, bei der die Betätigungswelle (8) drehbar und unverschieblich in einem Halteorgan (11) gehalten ist, das durch Führungsstangen (10a, 10b) undrehbar in bezug auf die Vorvakuumkammer (2) gehalten und mit dem Probenhalter (17) durch Haltestifte (14a,14b) verbindbar ist, die parallel zu der Betätigungswelle (8) verlaufen.

5. Vorrichtung nach einem der vorstehenden Ansprüche, bei der die Anschlußmittel (29a,29b,35;30a,30b,37a,37b) steckerförmige Anschlußteile bilden und die festen Anschlußmittel (35,37a,37b) in einer dem Sperrschieber (4) entgegengesetzten Position auf dem Gestell (15) angeordnet sind.

6. Vorrichtung nach einem der vorstehenden Ansprüche, bei der der Probenhalter einen Probentisch (24) aufweist, der in einem zentralen Bereich eines Isolators (23) versenkt ist, und bei der die Heizung (25) an einer Umfangsfläche des Isolators (23) angeordnet ist, um den Probentisch (24) zu beheizen.

## Revendications

1. Dispositif pour chauffer un échantillon (32) dans une chambre à vide (1) d'un analyseur ou analogue, comprenant:
- un plateau (15) pour échantillon disposé dans la chambre à vide,
- un porte-échantillon (17) disposé sur le plateau et pourvu d'un système chauffant (25),
- une chambre d'échappement préliminaire (2) reliée à la chambre à vide (1) via une vanne (4),
- un dispositif de transfert (6) comportant un arbre de manoeuvre (8) qui, à une première extrémité, est relié audit porte-échantillon (17) et peut coulisser longitudinalement pour enlever le porte-échantillon (17) dudit plateau (15) et l'amener dans la chambre d'échappement préliminaire (2) et pour ramener le porte-échantillon jusque dans la chambre à vide et l'installer sur le plateau,
- le porte-échantillon étant pourvu de moyens de connexion (29a,29b) qui coopèrent avec des moyens de connexion fixes (30a,30b) montés dans la chambre à vide pour alimenter électriquement le système chauffant (25) lorsque le porte-échantillon est monté sur le plateau,
**caractérisé** en ce que ledit arbre de manoeuvre (8) est relié au porte-échantillon (17) par l'intermédiaire d'un crochet (13) s'étendant perpendiculairement par rapport à l'arbre de manoeuvre (8) et pouvant s'introduire par une étroite ouverture (19) dans un trou d'enclenchement (20) du porte-échantillon (17), et l'arbre de manoeuvre (8) peut tourner autour de son axe longitudinal pour établir et supprimer l'enclenchement entre ledit crochet (13) et le porte-échantillon.

2. Dispositif selon la revendication 1, dans lequel le porte-échantillon (17) est pourvu d'un élément (26) de mesure de température et de moyens de connexion (30a,30b) qui coopèrent avec des moyens de connexion fixes (37a,37b) montés dans la chambre à vide (1) pour alimenter électriquement l'élément de mesure de température lorsque le porte-échantillon est monté sur le plateau.

3. Dispositif selon la revendication 1 ou 2, dans lequel ledit arbre de manoeuvre (8) est reçu dans un élément tubulaire faisant saillie vers l'extérieur depuis une paroi de la chambre d'échappement préliminaire (2), et un organe de manoeuvre (7) peut coulisser sur ledit élément tubulaire, ledit arbre de manoeuvre (8) étant entraîné grâce au fait qu'il est raccordé audit organe de manoeuvre (7) par un accouplement magnétique.

4. Dispositif selon l'une quelconque des revendications précédentes, dans lequel ledit arbre de manoeuvre (8) est retenu de manière à pouvoir tourner sans coulisser dans un élément de retenue (11) que des tiges de guidage (10a,10b) empêchent de tourner par rapport à la chambre d'échappement préliminaire (2) et qui peut être relié au porte-échantillon (17) par l'intermédiaire de doigts de retenue (14a,14b) qui s'étendent parallèlement à l'arbre de manoeuvre (8).

5. Dispositif selon l'une quelconque des revendications précédentes, dans lequel lesdits moyens de connexion (29a,29b,35;30a,30b,37a,37b) forment des connecteurs de type enfichable, lesdits moyens de connexion fixes (35,37a,37b) étant disposés sur le plateau (15) dans une position opposée à celle de la vanne (4).

6. Dispositif selon l'une quelconque des revendications précédentes, dans lequel ledit porte-échantillon comporte une table (24) à échantillon encastrée dans une partie centrale d'un élément isolant (23), ledit système chauffant (25) étant disposé sur une surface périphérique dudit élément isolant (23) pour chauffer la table (24) à échantillon.
